# EUROPÄISCHE PATENTSCHRIFT

(11) **EP 1 569 567 B1**
(45) Veröffentlichungstag und Bekanntmachung des Hinweises auf die Patenterteilung: **08.03.2006**
(21) Anmeldenummer: 03782310.1
(22) Anmeldetag: 05.12.2003
(51) Int. Cl.: A61B 17/17

(54) **FÜHRUNGSEINRICHTUNG FÜR EIN CHIRURGISCHES BEARBEITUNGSWERKZEUG**
GUIDE DEVICE FOR A SURGICAL MACHINING TOOL
DISPOSITIF DE GUIDAGE POUR UN INSTRUMENT DE TRAITEMENT CHIRURGICAL

(30) Priorität: 13.12.2002 DE 10258322
(43) Veröffentlichungstag der Anmeldung: 07.09.2005
(73) Patentinhaber: Aesculap AG & Co. KG, 78532 Tuttlingen (DE)
(72) Erfinder: BÖTTIGER, Roland, 78604 Rietheim-Weilheim (DE); RUTHE, Gerd, 78532 Tuttlingen (DE)
(74) Vertreter: Hoeger, Stellrecht & Partner Patentanwälte
(86) Internationale Anmeldenummer: PCT/EP2003/013747
(87) Internationale Veröffentlichungsnummer: WO 2004/054453

(56) Entgegenhaltungen:
- DE-C- 681 828
- DE-U- 20 202 615
- US-A- 5 190 547
- US-A- 5 228 459

## Beschreibung

Die Erfindung betrifft eine Führungseinrichtung für ein chirurgisches Bearbeitungswerkzeug mit einem das Bearbeitungswerkzeug führenden Führungselement, das an der Führungseinrichtung nur in einer Ebene verschiebbar gelagert ist.

Eine solche Führungseinrichtung ist beispielsweise aus der DE 202 02 615 U1 bekannt. Mit dieser Führungseinrichtung wird es möglich, ein Bearbeitungswerkzeug, beispielsweise einen schnellaufenden Fingerfräser, mittels einer Führungshülse so in einer Ebene zu führen, daß damit Knochenmaterial längs einer Ebene abgetragen werden kann. Gegebenenfalls kann durch Umsetzen von Teilen der Führungseinrichtung erreicht werden, daß auch in anderen Ebenen eine Bearbeitung des Knochens erfolgen kann, so daß beispielsweise das distale Ende eines Femurs für die Aufnahme einer entsprechenden Femurendoprothese vorbereitet werden kann. Bei der bekannten Vorrichtung wird das Führungselement in einer schlitzförmigen Führung der Führungseinrichtung geführt und kann in dieser schlitzförmigen Führung vom Benutzer hin- und hergeschoben und verschwenkt werden. Die schlitzförmige Führung kann in Einzelfällen dazu führen, daß die Sicht des Operateurs auf das Operationsgebiet beeinträchtigt werden könnte.

In der US 5,228,459 (Basis für den Oberbegriff des Anspruchs 1) ist eine solche Führungseinrichtung beschrieben, die über einen Lenker verschwenkbar mit einem Rahmen verbunden ist, so daß die Führungseinrichtung um eine Drehachse verschwenkt werden kann.

Es ist Aufgabe der Erfindung, eine gattungsgemäße Führungseinrichtung so auszubilden, daß eine Bewegung des Führungselementes in der Ebene mög-lich ist, die keine reine Schwenkbewegung ist, sondern die unterschiedlich und variabel ausgestaltet werden kann.

Diese Aufgabe wird bei einer Führungseinrichtung der eingangs beschriebenen Art erfindungsgemäß dadurch gelöst, daß an einem Rahmen der Führungseinrichtung zwei Lenker mittels eines Drehgelenkes um parallel zueinander und senkrecht auf der Führungsebene stehende Drehachsen verdrehbar gelagert sind, die ihrerseits mit dem Führungselement um parallel zu diesen Drehachsen verlaufende Drehachsen verdrehbar verbunden sind.

Das Führungselement erfährt somit über zwei Lenker oder Schwenkhebel eine sehr zuverlässige, weitgehend spielfreie Lagerung, so daß sichergestellt ist, daß das Bearbeitungswerkzeug auch bei robustem Einsatz exakt in der Führungsebene geführt wird.

Die Verbindungslinien der beiden Drehgelenke der beiden Lenker können parallel zueinander verlaufen, dann ergibt sich eine Parallelogrammführung, bei der das Führungselement parallel zu sich selbst auf einer Kreisbahn verschoben wird, deren Radius durch den Drehachsenabstand der Lenker definiert wird. Bei einer anderen Ausführungsform kann jedoch vorgesehen sein, daß die Verbindungslinien der beiden Drehgelenke der beiden Lenker schräg zueinander verlaufen. Dies führt beim Verschwenken der Lenker zu einer gleichzeitigen Verschwenkung des Führungselementes, wobei diese Verschwenkbewegung davon abhängig ist, wie groß der Winkel der beiden Lenker zueinander ist.

Günstig ist es, wenn das Führungselement in der Führungsebene zwei abgewinkelt zueinander verlaufende Schenkel umfaßt, insbesondere kann der Winkel zwischen den beiden Schenkeln zwischen 75° und 105° liegen, vorzugsweise bei etwa 90°. Durch diese Anordnung ist es möglich, das Führungselement aus der Verbindungslinie der Lagerung des Führungselementes an den Lenkern hervorstehen zu lassen, man kann auf diese Weise relativ lange Lenker verwenden und trotzdem die Führung des Bearbeitungswerkzeuges in Richtung auf die Bearbeitungsstelle verlagern.

Das Führungselement selbst kann das Bearbeitungswerkzeug in beliebiger Weise führen, besonders vorteilhaft ist es, wenn das Führungselement eine das Bearbeitungswerkzeug aufnehmende Führungshülse trägt.

Diese Führungshülse kann fest mit dem Führungselement verbunden sein, es ist aber auch möglich, daß die Führungshülse um eine Drehachse verdrehbar an dem Führungselement gelagert ist, die parallel zur Drehachse des Lenkers oder der Lenker verläuft. Auf diese Weise ist die Benutzungsperson frei, die Führungshülse und damit das Bearbeitungswerkzeug in der Führungsebene in beliebige Richtung zu verschwenken.

Wenn gemäß einer bevorzugten Ausführungsform die Längsachse der Führungshülse in der Führungsebene liegt, läßt sich mit dem Bearbeitungswerkzeug eine Fläche bearbeiten, die parallel zur Führungsebene verläuft. Grundsätzlich wäre es auch möglich, die Längsachse der Führungshülse gegenüber der Führungsebene zu neigen, so daß dann die bearbeitete Fläche gegenüber der Führungsebene ebenfalls geneigt ist.

Die Führungshülse kann bei einem abgewinkelten Führungselement vorzugsweise an der Verbindungsstelle der beiden Schenkel an dem Führungselement gehalten sein, so daß die Führungshülse möglichst dicht an der Bearbeitungsstelle positioniert werden kann.

Es ist vorteilhaft, wenn die Verschwenkmöglichkeit des Lenkers oder der Lenker durch einen Anschlag eingeschränkt ist, der vorzugsweise in seiner Position verstellbar ist.

Der oder die Lenker oder auch das Führungselement können Durchbrüche aufweisen, insbesondere in Form von Längsschlitzen. Dadurch wird einerseits ihr Gewicht herabgesetzt, zum anderen ergeben sich für den Operateur Sichtfelder, durch diese Durchbrüche hindurch kann er das Operationsgebiet beobachten.

Bei einer einfachen Ausgestaltung der Führungseinrichtung läßt sich das Führungselement durch den Lenker oder die Lenker in einer Führungsebene bewegen. Wenn die Bearbeitung des Knochens in unterschiedlichen Ebenen gewünscht wird, insbesondere in unterschiedlich gerichteten Ebenen, wie dies beispielsweise bei der Vorbereitung eines distalen Femurs der Fall ist, dann ist es vorteilhaft, wenn der oder die Lenker am Rahmen um verschiedene Drehachsen verschwenkbar lagerbar sind. Hierzu gibt es unterschiedliche Realisierungsmöglichkeiten, beispielsweise kann die Lage der Drehgelenke des oder der Lenker am Rahmen verstellbar sein. Die Drehgelenke können an einem Teil des Rahmens gelagert sein, welcher gegenüber der Gesamtführungseinrichtung z. B. verschwenkbar ist.

Bei einer andere Ausgestaltung ist vorgesehen, daß mehrere Drehgelenkstellen mit unterschiedlichen Drehachsen am Rahmen angeordnet sind, an denen der oder die Lenker wahlweise lagerbar sind.

Bei einer weiteren Gestaltungsmöglichkeit sind der oder die Lenker mit ihren Drehgelenken in verschiedene Aufnahmen des Rahmens einsetzbar.

Es kann auch vorgesehen sein, daß die rahmenseitigen Teile des Drehgelenkes des oder der Lenker in unterschiedlicher Lage in dieselben Aufnahmen am Rahmen einsetzbar sind.

In allen Fällen wird erreicht, daß am selben Rahmen und damit an derselben Führungseinrichtung die Lenker in unterschiedlichen Positionen und in unterschiedlichen Orientierungen gelagert werden können, so daß sich auch unterschiedliche Führungsebenen ergeben, die an die gewünschten Bearbeitungsflächen angepaßt sind. Es ist damit möglich, auch komplizierte Bearbeitungsvorgänge an einem Knochen mit nur einer Führungseinrichtung zu bewerkstelligen, an der das Führungselement mittels der Lenker in unterschiedlichen Ebenen verschwenkbar aufgenommen wird.

Die nachfolgende Beschreibung bevorzugter Ausführungsformen der Erfindung dient im Zusammenharig mit der Zeichnung der näheren Erläuterung. Es zeigen :
- Figur 1:: eine perspektivische Ansicht einer modulartig aufgebauten, am distalen Femur gehaltenen Führungseinrichtung für ein fingerfräserartiges Bearbeitungswerkzeug vor dem Zusammenfügen der Module der Führungseinrichtung;
- Figur 2:: eine Querschnittsansicht des Rahmenteils der Führungseinrichtung der Figur 1 mit dem Führungselement in einer ersten Position;
- Figur 3:: eine Ansicht ähnlich Figur 2 mit dem Führungselement in einer zweiten Position;
- Figur 4:: eine schematische Ansicht ähnlich Figur 3 mit dem Führungselement in einer dritten Position;
- Figur 5:: eine Ansicht ähnlich Figur 3 mit dem Führungselement in einer vierten Position;
- Figur 6:: eine Ansicht ähnlich Figur 2 mit dem Führungselement in einer fünften Position;
- Figur 7:: eine Draufsicht auf die Lenker, das Führungselement und das darin geführte Bearbeitungswerkzeug in einer Mittelstellung;
- Figur 8:: eine Schnittansicht längs Linie 8-8 in Figur 7;
- Figur 9:: eine Ansicht ähnlich Figur 7 in einer aus der Mittelstellung herausgeschwenkten Stellung des Führungselementes;
- Figur 10:: eine Ansicht ähnlich Figur 7 bei einem anderen bevorzugten Ausführungsbeispiel einer Führungseinrichtung;
- Figur 11:: eine Schnittansicht längs Linie 11-11 in Figur 10 und
- Figur 12:: eine Ansicht ähnlich Figur 10 mit einem aus der Mittelstellung herausgeschwenkten Führungselement.

Die Erfindung wird erläutert anhand einer Vorrichtung, wie sie zur Vorbereitung des distalen Femurs Verwendung findet, es versteht sich aber von selbst, daß die beschriebene Einrichtung auch bei der Bearbeitung anderer Knochen des Körpers eingesetzt werden kann, bei denen es von Bedeutung ist, daß die Knochen längs einer Ebene bearbeitet werden, also beim Anbringen von Sägeschnitten oder auch bei der Bearbeitung einer Fläche mittels eines schnell laufenden Fingerfräsers.

Zur Festlegung der neuen Führungseinrichtung 1 am distalen Femur 2 können sehr unterschiedliche Vorrichtungen verwendet werden, beim vorliegenden Ausführungsbeispiel wird die Führungseinrichtung 1 mittels einer Haltevorrichtung 3 am distalen Femur 2 festgelegt, die modular aufgebaut ist und eine Plattform 4 umfaßt, die über pfostenähnliche Stifte 5 am Femur 2 gehalten ist. Auf diese Plattform 4 kann ein Justierungsmodul 6 aufgeschoben werden, an dem ein im wesentlichen U-förmiger Haltebügel 7 angeordnet ist, dieser kann über unterschiedliche, hier nicht näher erläuterte Stellglieder 8 relativ zum Femur 2 in der gewünschten Weise ausgerichtet werden. Insofern entspricht die Haltevorrichtung 3 der Haltevorrichtung, die beispielsweise in der DE 202 02 615 U1 beschrieben ist und auf die ausdrücklich Bezug genommen wird.

Der Haltebügel 7 weist einen horizontalen Steg 9 und zwei senkrecht davon abstehende Schenkel 10, 11 auf, an den freien Enden der Schenkel 10, 11 sind parallel zum Steg 9 verlaufende, nutförmige Aufnahmen 12 angeordnet, in die von der Seite her Lagerböcke 13 geführt einschiebbar sind. Diese Lagerböcke 13 können mit Klemmplatten 14 in den Aufnahmen 12 fixiert werden, die Klemmplatten 14 werden durch Klemmschrauben 15 gespannt.

Die beschriebene Haltevorrichtung 3 mit dem Haltebügel 7 bildet einen Rahmen aus, an dem die eigentliche Führungseinrichtung 1 gelagert wird, und zwar über die Lagerböcke 13.

Diese Führungseinrichtung 1 umfaßt zwei stabförmige Lenker 16, 17, die jeweils an einem Ende drehbar an jeweils einem Lagerbock 13 gelagert sind. Die Drehachse des entsprechenden Drehgelenks 18 verläuft parallel zu den Schenkeln 10, 11 und liegt nahe bei diesen Schenkeln 10, 11, da die Lagerböcke 13 nur relativ wenig aus den nutförmigen Aufnahmen 12 hervorstehen.

Beide Lenker 16, 17 sind geradlinig und plattenförmig ausgebildet, in dem einen der beiden Lenker befindet sich ein Durchbruch 19 in Form eines Längsschlitzes, grundsätzlich könnten beide Lenker mit oder ohne Durchbruch ausgebildet sein.

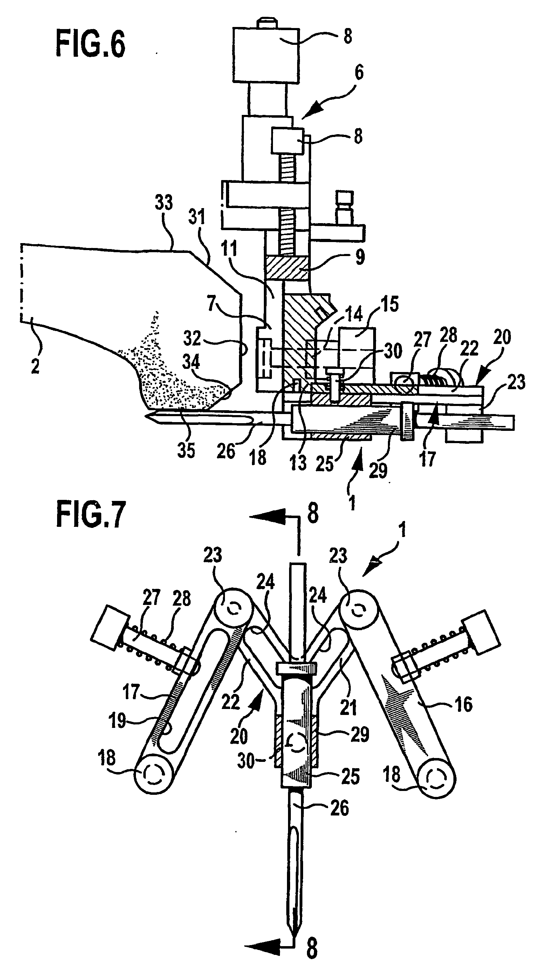

An dem dem Drehgelenk 18 abgewandten Ende sind beide Lenker 16, 17 verschwenkbar mit einem Führungselement 20 verbunden, welches als zweiarmiger Hebel mit zwei Hebelarmen 21, 22 ausgebildet ist, die miteinander einen Winkel von etwa 90° einschließen. Die dadurch ausgebildeten Drehgelenke 23 weisen Drehachsen auf, die parallel zu den Drehachsen der Drehgelenke 18 verlaufen, die Drehgelenke 23 sind an den freien Enden der Lenker 16, 17 bzw. der Hebelarme 21, 22 positioniert. Die Länge der Hebelarme 21, 22 ist so gewählt, daß der Abstand der Drehgelenke 23 voneinander kleiner ist als der Abstand der Drehgelenke 18 voneinander, die Lenker 16, 17 verlaufen also schräg zueinander (Figur 7). In den Hebelarmen 21, 22 sind ebenfalls längsschlitzartige Durchbrüche 24 vorgesehen.

An der Verbindungsstelle der beiden Hebelarme 21, 22 ist an einer Seite des plattenförmigen Führungselementes 20 eine Führungshülse 25 gehalten. Diese Halterung kann eine feste Halterung sein, es ist aber auch möglich, die Führungshülse 25 in eine Aufnahmeöffnung einzuschieben und sie dort durch Reibungskräfte lösbar zu fixieren. Die Führungshülse 25 dient der Aufnahme des Schaftes eines schnellaufenden Fingerfräsers 26, die Führungshülse 25 kann dazu besonders ausgestaltet sein, beispielsweise durch am Schaft des Fingerfräsers 26 anliegende Kugellager. Dies wird hier nicht näher erläutert.

Bei einer ersten Ausgestaltung ist die Führungshülse 25 starr mit dem Führungselement 20 verbunden, die Längsachse der Führungshülse 25 verläuft dann auf der Winkelhalbierenden der beiden Hebelarme 21, 22.

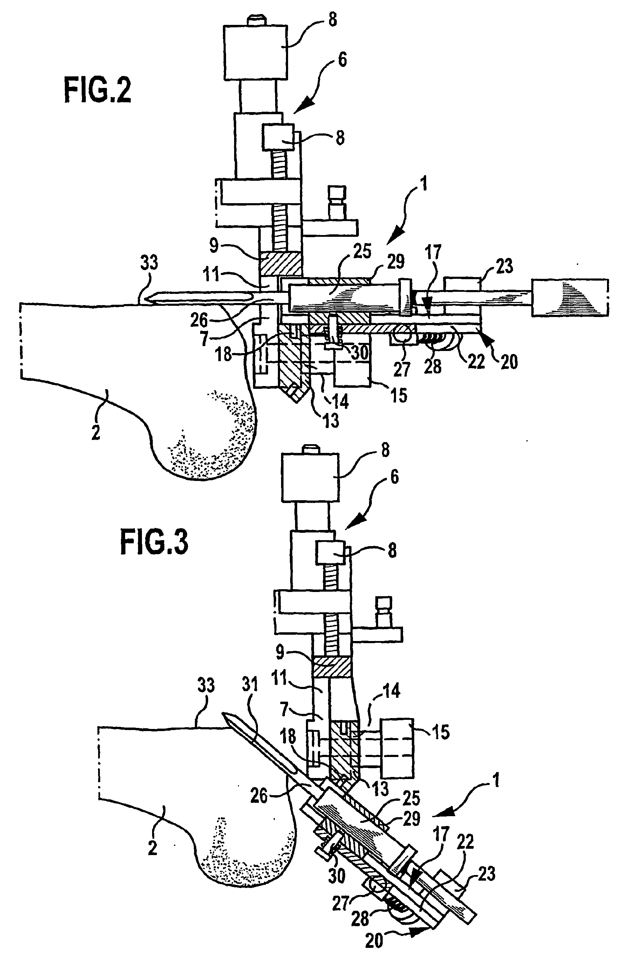

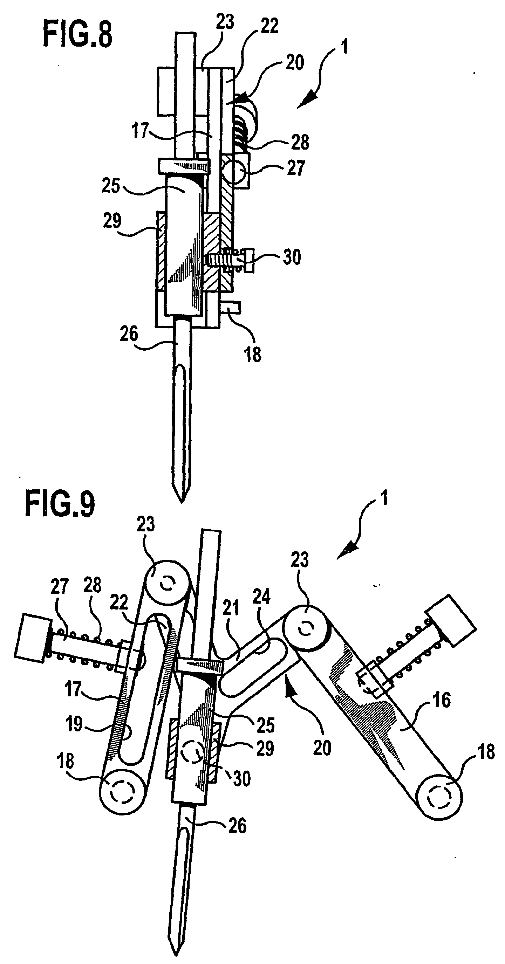

Der Operateur kann bei der beschriebenen Führungseinrichtung 1 den Fingerfräser 26 in die Führungshülse 25 einschieben und dann durch Verschwenken des Fingerfräsers 26 in einer Ebene, die parallel zum Führungselement 20 verläuft, die Führungshülse 25 in dieser Ebene, die nachfolgend Führungsebene genannt wird, geführt verschwenken. Die Führung erfolgt dabei durch die beiden Lenker 16, 17, die aufgrund ihres zueinander schrägen Verlaufes beim Verschwenken auch das Führungselement 20 verschwenken, diese Verschwenkung des Führungselementes 20 hängt von den Längenverhältnissen und von der Schrägstellung der beiden Hebelarme 21, 22 zueinander ab. In Figur 7 ist die Führungseinrichtung 1 in einer Mittelstellung dargestellt, in Figur 9 in einer verschwenkten Stellung. Durch diese geführte Verschwenkung überstreicht der Fingerfräser 26 definiert einen bestimmten Bereich in der Führungsebene und kann diesen Bereich bearbeiten, beispielsweise an der dorsalen Seite des distalen Femurs, wie dies in Figur 2 dargestellt ist. Der Schwenkbereich wird in diesem Falle durch die Führungshülse 25 vorgegeben, er läßt sich dadurch beschränken, daß die Schwenkbewegung der Hebelarme 21, 22 eingeschränkt wird. Zu diesem Zweck tragen die Lenker 16, 17 verstellbare Anschlagstifte 27, die quer zur Längsrichtung der Lenker 16, 17 an diesen gehalten sind und mehr oder weniger tief eingeschoben werden können, so daß sie bei der Verschwenkung der Lenker 16, 17 an dem Führungselement 20 anschlagen und dadurch die Bewegungsmöglichkeit der Führungshülse 25 und damit die von dem Fingerfräser 26 überstrichene Fläche einschränken. Die Anschlagstifte 27 sind in der Zeichnung alle in unwirksamer, herausgezogener Stellung gezeigt, sie können aber gegen die Wirkung von sie umgebenden Schraubenfedem 28 eingeschoben werden.

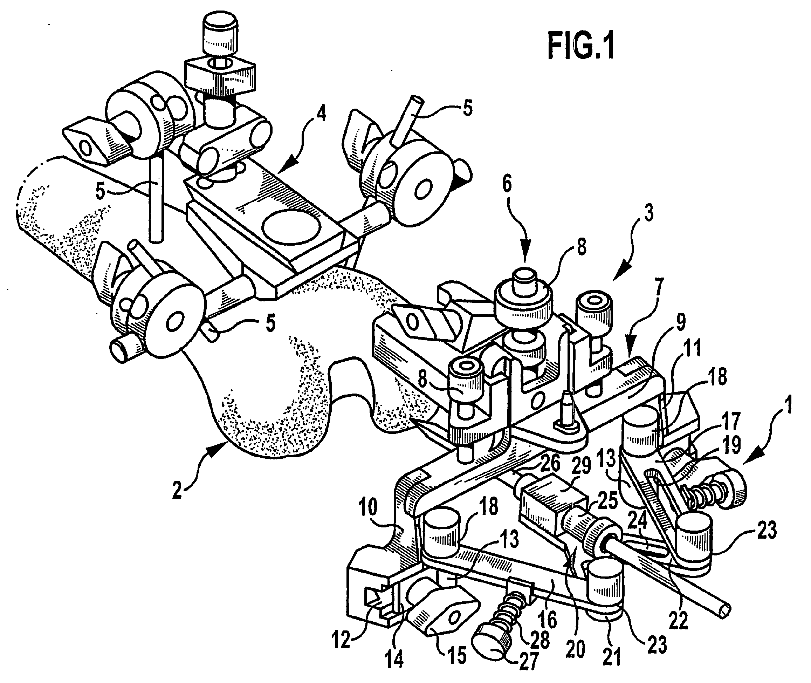

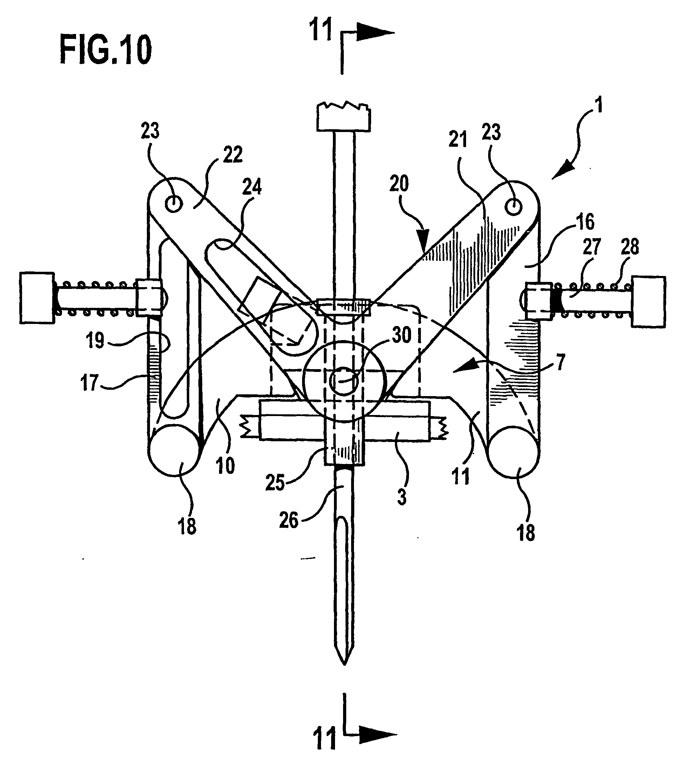

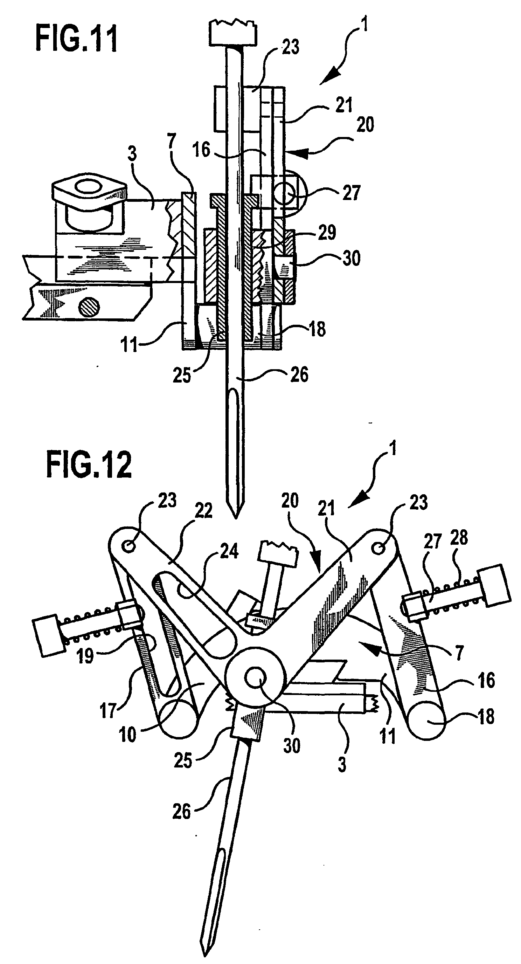

Während bei dem Ausführungsbeispiel der Figuren 1 bis 9 die Lenker 16, 17 schräg zueinander verlaufen, ist bei dem Ausführungsbeispiel der Figuren 10 bis 12 ein Verlauf der Lenker 16, 17 gewählt, bei dem diese immer parallel zueinander angeordnet sind, d. h. der Abstand der Drehgelenke 23 voneinander ist genau gleich wie der Abstand der Drehgelenke 18 voneinander.

Dadurch wird das Führungselement 20 immer parallel zu sich bewegt, wenn die Lenker 16, 17 verschwenkt werden.

Die Führungshülse 25 kann am Führungselement 20 um eine Drehachse verschwenkbar gelagert sein, die parallel zu den Drehachsen der Drehgelenke 18 und 23 verläuft, so daß die Führungshülse 25 in der Führungsebene nicht nur zusammen mit dem Führungselement 20 verschoben werden kann, sondern die Führungshülse 25 kann in der Führungsebene zusätzlich um diese Drehachse relativ zum Führungselement 20 verdreht werden. Zu diesem Zweck ist ein die Führungshülse 25 aufnehmender Lagerblock 29 mittels eines Lagerstiftes 30 im Verbindungsbereich der Hebelarme 21 und 22 drehbar gelagert.

Bei der in den Figuren 1 und 2 dargestellten Anordnung ergibt sich eine bestimmte Führungsebene, d. h. in dieser Anordnung läßt sich mit der Führungseinrichtung 1 eine einzige Fläche am Femur 2 bearbeiten, in dem Fall der Figuren 1 und 2 am distalen Femur.

Um andere Flächen bearbeiten zu können, ist vorgesehen, daß die Führungseinrichtung 1 an der Haltevorrichtung 3 in unterschiedlichen Positionen festgelegt werden kann.

Dies läßt sich beispielsweise dadurch erreichen, daß an den Lagerböcken 13 unterschiedliche Lagerungsmöglichkeiten für die Lenker 16, 17 vorgesehen sind, beispielsweise gemäß der Darstellung der Figur 3 am oberen Ende des Lagerbockes 13 und mit geneigter Drehachse am unteren Ende des Lagerbockes 13. Es ist dadurch möglich, die Führungseinrichtung 1 unter einem der Neigung dieser Drehachse entsprechenden Winkel an der Haltevorrichtung festzulegen und damit eine geneigte Führungsebene zu erzeugen, durch die eine schräge Fläche 31 am Femur bearbeitet werden kann.

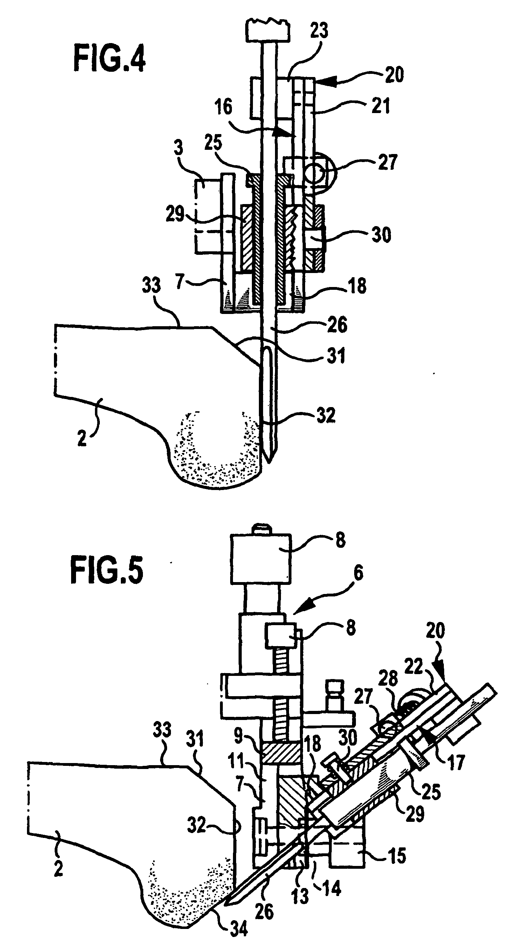

Es können unterschiedliche Lagerböcke 13 eingesetzt werden, so daß weitere Orientierungen möglich sind, in Figur 4 ist beispielsweise eine Orientierung dargestellt, mit der eine distale Femurfläche 32 bearbeitet werden kann, die senkrecht auf der dorsalen Fläche 33 steht, die bei der Anordnung der Figuren 1 und 2 erreichbar ist, bei der Ausgestaltung gemäß Figur 5 läßt sich eine schräge anteriore Fläche 34 bearbeiten, mit der Anordnung gemäß Figur 6 eine senkrecht zur distalen Fläche 32 stehende anteriore Fläche 35.

Dabei ist es möglich, die Führungseinrichtung 1 dauerhaft mit den zugehörigen Lagerböcken 13 zu verbinden und diese zusammen mit einer Führungseinrichtung 1 auszuwechseln, wenn andere Orientierungen gewünscht sind. Es ist aber auch möglich, die Führungseinrichtung 1 jeweils mit anderen Lagerböcken 13 zu verbinden oder andere Lagerstellen an diesen Lagerböcken 13 zu verwenden, um so mit einer einzigen Führungseinrichtung 1 und unterschiedlichen Lagerböcken 13 die verschiedenen Orientierungen zu erreichen.

Bei einer anderen, in der Zeichnung nicht dargestellten Ausführungsform wäre es auch möglich, die Führungseinrichtung 1 verstellbar auszubilden, beispielsweise könnten die Haltebügel 7 verschwenkbar ausgebildet sein, hier sind unterschiedliche Einstellmöglichkeiten denkbar.

Durch die Lagerung der Führungshülse 25 an dem Rahmen unter Verwendung der Lenker 16, 17 ergibt sich eine sehr sichere Führung der Führungshülse 25, die weitgehend spielfrei möglich ist und die dem Operateur eine zuverlässige Führung der Führungshülse in der Führungsebene und längs einer genau definierten Verschiebebahn ermöglicht, dies erhöht die Sicherheit beim Bearbeitungsvorgang und gibt aufgrund der speziellen Konstruktion dem Operateur darüber hinaus die Möglichkeit, den Operationsbereich während des gesamten Bearbeitungsvorganges beobachten zu können.

## Patentansprüche

1. Führungseinrichtung (1) für ein chirurgisches Bearbeitungswerkzeug (26) mit einem das Bearbeitungswerkzeug (26) führenden Führungselement (20), das an der Führungseinrichtung (1) nur in einer Ebene verschiebbar gelagert ist, **dadurch gekennzeichnet, daß** an einem Rahmen (3, 6) der Führungseinrichtung (1) zwei Lenker (16, 17) mittels eines Drehgelenkes (18) um parallel zueinander und senkrecht auf der Führungsebene stehende Drehachsen verdrehbar gelagert sind, die ihrerseits mit dem Führungselement (20) um parallel zu diesen Drehachsen verlaufende Drehachsen verdrehbar verbunden sind.

2. Führungseinrichtung nach Anspruch 1, **dadurch gekennzeichnet, daß** die Verbindungslinien der beiden Drehgelenke (18, 23) der beiden Lenker (16, 17) parallel zueinander verlaufen.

3. Führungseinrichtung nach Anspruch 1, **dadurch gekennzeichnet, daß** die Verbindungslinien der beiden Drehgelenke (18, 23) der beiden Lenker (16, 18) schräg zueinander verlaufen.

4. Führungseinrichtung nach einem der voranstehenden Ansprüche, **dadurch gekennzeichnet, daß** das Führungselement (20) in der Führungsebene (2) abgewinkelt zueinander verlaufende Schenkel (21, 22) umfaßt.

5. Führungseinrichtung nach Anspruch 4, **dadurch gekennzeichnet, daß** der Winkel zwischen den beiden Schenkeln (21, 22) zwischen 75° und 105° liegt.

6. Führungseinrichtung nach einem der voranstehenden Ansprüche, **dadurch gekennzeichnet, daß** das Führungselement (20) eine das Bearbeitungswerkzeug (26) aufnehmende Führungshülse (25) trägt.

7. Führungseinrichtung nach Anspruch 6, **dadurch gekennzeichnet, daß** die Führungshülse (25) fest mit dem Führungselement (20) verbunden ist.

8. Führungseinrichtung nach Anspruch 6, **dadurch gekennzeichnet, daß** die Führungshülse (25) um eine Drehachse verdrehbar an dem Führungselement (20) gelagert ist, die parallel zur Drehachse des Lenkers oder der Lenker (16, 17) verläuft.

9. Führungseinrichtung nach einem der Ansprüche 6 bis 8, **dadurch gekennzeichnet, daß** die Längsachse der Führungshülse (25) in der Führungsebene liegt.

10. Führungseinrichtung nach einem der Ansprüche 6 bis 9 und nach einem der Ansprüche 5 oder 6, **dadurch gekennzeichnet, daß** die Führungshülse (25) an der Verbindungsstelle der beiden Schenkel (21, 22) an dem Führungselement (20) gehalten ist.

11. Führungseinrichtung nach einem der voranstehenden Ansprüche, **dadurch gekennzeichnet, daß** die Verschwenkmöglichkeit des Lenkers oder der Lenker (16, 17) durch einen Anschlag (27) eingeschränkt ist.

12. Führungseinrichtung nach Anspruch 11, **dadurch gekennzeichnet, daß** der Anschlag (27) in seiner Position verstellbar ist.

13. Führungseinrichtung nach einem der voranstehenden Ansprüche, **dadurch gekennzeichnet, daß** der oder die Lenker (16, 17) und/oder das Führungselement (20) Durchbrüche (19; 24) aufweisen.

14. Führungseinrichtung nach Anspruch 13, **dadurch gekennzeichnet, daß** die Durchbrüche (19; 24) als Längsschlitze ausgebildet sind.

15. Führungseinrichtung nach einem der voranstehenden Ansprüche, **dadurch gekennzeichnet, daß** der oder die Lenker (16, 17) am Rahmen (3, 6) um verschiedene Drehachsen verschwenkbar lagerbar sind.

16. Führungseinrichtung nach Anspruch 15, **dadurch gekennzeichnet, daß** die Lage der Drehgelenke (18) des oder der Lenker (16, 17) am Rahmen (3, 6) verstellbar ist.

17. Führungseinrichtung nach Anspruch 15, **dadurch gekennzeichnet, daß** mehrere Drehgelenkstellen mit unterschiedlichen Drehachsen am Rahmen (3, 6) angeordnet sind, an denen der oder die Lenker (16, 17) wahlweise lagerbar sind.

18. Führungseinrichtung nach Anspruch 15, **dadurch gekennzeichnet, daß** der oder die Lenker (16, 17) mit ihrem Drehgelenk (18) in verschiedene Aufnahmen (12) des Rahmens (3, 6) einsetzbar sind.

19. Führungseinrichtung nach Anspruch 15, **dadurch gekennzeichnet, daß** die rahmenseitigen Teile des Drehgelenkes (18) des oder der Lenker (16, 17) in unterschiedlicher Lage in dieselbe Aufnahme (12) am Rahmen (3, 6) einsetzbar sind.

## Claims

1. Guiding device (1) for a surgical machining tool (26) with a guiding element (20) which guides the machining tool (26) and is mounted on the guiding device (1) so as to be displaceable in one plane only, **characterized in that** two levers (16, 17) are mounted on a frame (3, 6) of the guiding device (1) by means of a pivot (18) so as to be rotatable about axes of rotation extending parallel to each other and perpendicularly to the guiding plane, and the said levers are, in turn, connected to the guiding element (20) so as to be rotatable about axes of rotation extending parallel to these axes of rotation.

2. Guiding device in accordance with claim 1, **characterized in that** the lines connecting the two pivots (18, 23) of the two levers (16, 17) extend parallel to each other.

3. Guiding device in accordance with claim 1, **characterized in that** the lines connecting the two pivots (18, 23) of the two levers (16, 18) extend at an incline to each other.

4. Guiding device in accordance with any one of the preceding claims, **characterized in that** the guiding element (20) comprises legs (21, 22) extending at an angle to each other in the guiding plane (2).

5. Guiding device in accordance with claim 4, **characterized in that** the angle between the two legs (21, 22) lies between 75° and 105°.

6. Guiding device in accordance with any one of the preceding claims, **characterized in that** the guiding element (20) carries a guide sleeve (25) which receives the machining tool (26).

7. Guiding device in accordance with claim 6, **characterized in that** the guide sleeve (25) is fixedly connected to the guiding element (20).

8. Guiding device in accordance with claim 6, **characterized in that** the guide sleeve (25) is mounted on the guiding element (20) so as to be rotatable about an axis of rotation which extends parallel to the axis of rotation of the lever or levers (16, 17).

9. Guiding device in accordance with any one of claims 6 to 8, **characterized in that** the longitudinal axis of the guide sleeve (25) lies in the guiding plane.

10. Guiding device in accordance with any one of claims 6 to 9 and in accordance with claim 5 or 6, **characterized in that** the guide sleeve (25) is held at the junction of the two legs (21, 22) on the guiding element (20).

11. Guiding device in accordance with any one of the preceding claims, **characterized in that** the pivotability of the lever or levers (16, 17) is limited by a stop (27).

12. Guiding device in accordance with claim 11, **characterized in that** the stop (27) is adjustable in its position.

13. Guiding device in accordance with any one of the preceding claims, **characterized in that** the lever or levers (16, 17) and/or the guiding element (20) have openings (19; 24).

14. Guiding device in accordance with claim 13, **characterized in that** the openings (19; 24) are formed as elongate slots.

15. Guiding device in accordance with any one of the preceding claims, **characterized in that** the lever or levers (16, 17) are mountable on the frame (3, 6) so as to be pivotable about various axes of rotation.

16. Guiding device in accordance with claim 15, **characterized in that** the position of the pivots (18) of the lever or levers (16, 17) on the frame (3, 6) is adjustable.

17. Guiding device in accordance with claim 15, **characterized in that** several pivot locations with different axes of rotation are arranged on the frame (3, 6) for optional mounting of the lever or levers (16, 17) thereat.

18. Guiding device in accordance with claim 15, **characterized in that** the lever or levers (16, 17) is or are insertable with its or their pivot (18) in various receivers (12) of the frame (3, 6).

19. Guiding device in accordance with claim 15, **characterized in that** those parts of the pivot (18) of the lever or levers (16, 17) that are arranged on the frame are insertable in a different position into the same receiver (12) on the frame (3, 6).

## Revendications

1. Dispositif de guidage (1) pour un outil d'usinage chirurgical (26), comprenant un élément de guidage (20) qui guide l'outil d'usinage (26) et qui est monté sur le dispositif de guidage (1) en n'étant coulissant que dans un seul plan, **caractérisé en ce que** sur un châssis (3, 6) du dispositif de guidage (1) sont montées, chacune au moyen d'une articulation de rotation (18), deux biellettes (16, 17) de façon à pouvoir ainsi tourner autour d'axes de rotation mutuellement parallèles et perpendiculaires au plan de guidage, ces biellettes étant pour leur part reliées à l'élément de guidage (20) en pouvant tourner autour d'axes de rotation s'étendant parallèlement aux axes de rotation cités précédemment.

2. Dispositif de guidage selon la revendication 1, **caractérisé en ce que** les lignes de jonction des deux articulations (18, 23) des deux biellettes (16, 17) s'étendent parallèlement l'une à l'autre.

3. Dispositif de guidage selon la revendication 1, **caractérisé en ce que** les lignes de jonction des deux articulations (18, 23) des deux biellettes (16, 17) s'étendent de manière oblique l'une par rapport à l'autre.

4. Dispositif de guidage selon l'une des revendications précédentes, **caractérisé en ce que** l'élément de guidage (20) comprend des branches (21, 22) qui s'étendent de manière coudée l'une par rapport à l'autre dans le plan de guidage (2).

5. Dispositif de guidage selon la revendication 4, **caractérisé en ce que** l'angle entre les deux branches (21, 22) se situe entre 75° et 105°.

6. Dispositif de guidage selon l'une des revendications précédentes, **caractérisé en ce que** l'élément de guidage (20) porte un fourreau de guidage (25) recevant l'outil d'usinage (26).

7. Dispositif de guidage selon la revendication 6, **caractérisé en ce que** le fourreau de guidage (25) est lié de manière fixe à l'élément de guidage (20).

8. Dispositif de guidage selon la revendication 6, **caractérisé en ce que** le fourreau de guidage (25) est monté sur l'élément de guidage (20) de manière à pouvoir tourner autour d'un axe de rotation qui s'étend parallèlement à l'axe de rotation de la biellette ou des biellettes (16, 17).

9. Dispositif de guidage selon l'une des revendications 6 à 8, **caractérisé en ce que** l'axe longitudinal du fourreau de guidage (25) se situe dans le plan de guidage.

10. Dispositif de guidage selon l'une des revendications 6 à 9 et selon l'une des revendications 5 ou 6, **caractérisé en ce que** le fourreau de guidage (25) est maintenu sur l'élément de guidage (20) au niveau de la zone de liaison des deux branches (21, 22).

11. Dispositif de guidage selon l'une des revendications précédentes, **caractérisé en ce que** la possibilité de pivotement de la biellette ou des biellettes (16, 17) est limitée par une butée (27).

12. Dispositif de guidage selon la revendication 11, **caractérisé en ce que** la butée (27) est réglable quant à sa position.

13. Dispositif de guidage selon l'une des revendications précédentes, **caractérisé en ce que** la biellette ou les biellettes (16, 17) et/ou l'élément de guidage (20) présentent des évidements de passage (19; 24).

14. Dispositif de guidage selon la revendication 13, **caractérisé en ce que** les évidements de passage (19 ; 24) sont réalisés sous la forme de fentes longitudinales.

15. Dispositif de guidage selon l'une des revendications précédentes, **caractérisé en ce que** la biellette ou les biellettes (16, 17) peuvent être montées sur le châssis (3, 6) de manière à pouvoir pivoter autour de différents axes de rotation.

16. Dispositif de guidage selon la revendication 15, **caractérisé en ce que** la position des articulations de rotation (18) de la biellette ou des biellettes (16, 17) sur le châssis (3, 6), est réglable.

17. Dispositif de guidage selon la revendication 15, **caractérisé en ce que** sur le châssis (3, 6) sont agencés plusieurs points d'articulation de rotation avec différents axes de rotation, sur lesquels, au choix, peuvent être montés la biellette ou les biellettes (16, 17).

18. Dispositif de guidage selon la revendication 15, **caractérisé en ce que** la biellette ou les biellettes (16, 17) peuvent être insérées, avec leur articulation de rotation (18), dans différents logements de réception (12) du châssis (3, 6).

19. Dispositif de guidage selon la revendication 15, **caractérisé en ce que** les parties, côté châssis, de l'articulation de rotation (18) de la biellette ou des biellettes (16, 17), peuvent être insérées dans des positions différentes dans le même logement de réception (12) sur le châssis (3, 6).
